(19) Europäisches Patentamt
European Patent Office
Office européen des brevets

(11) Numéro de publication: **0 063 084**
**B1**

# (12) FASCICULE DE BREVET EUROPEEN

(45) Date de publication du fascicule du brevet: **12.09.84**

(51) Int. Cl.³: **C 07 D 211/14**, A 61 K 31/445

(21) Numéro de dépôt: **82400652.2**

(22) Date de dépôt: **09.04.82**

(54) **Dérivés de phénéthanolamine, leur préparation et leur application en thérapeutique.**

(30) Priorité: **14.04.81 FR 8107445**

(43) Date de publication de la demande:
**20.10.82 Bulletin 82/42**

(45) Mention de la délivrance du brevet:
**12.09.84 Bulletin 84/37**

(84) Etats contractants désignés:
**AT BE CH DE FR GB IT LI LU NL SE**

(56) Documents cités:
**DE - A - 1 618 010**
**FR - A - 2 070 102**
**FR - A - 2 371 436**
**FR - M - 5 733**

(73) Titulaire: **SYNTHELABO, 58, rue de la Glacière, F-75621 Paris Cedex 13 (FR)**

(72) Inventeur: **Wick, Alexander, 10 Boulevard des Plants, F-78800 Saint Nom La Breteche (FR)**
Inventeur: **Frost, Jonathan, 15, rue des Amandiers, F-92340 Cachan (FR)**

(74) Mandataire: **Ludwig, Jacques et al, SYNTHELABO Service Brevets 58, rue de la Glacière, F-75621 Paris Cedex 13 (FR)**

**EP 0 063 084 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés de phénéthanolamine, leur préparation et leur application en thérapeutique.

Les composés de l'invention, sous forme de base libre, répondent à la formule (I)

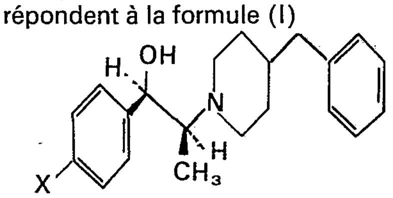

(I)

dans laquelle X est un atome d'halogène. Leurs sels d'addition à des acides acceptables du point de vue pharmacologique font également partie de l'invention.

Les composés de l'invention sont obtenus sous la forme (±)érythro.

Selon l'invention on peut préparer les composés (I) selon le schéma réactionnel donné ci-après.

On fait réagir une halogénure-4 propiophénone de formule (II) avec du brome dans un solvant tel que le chloroforme pour obtenir le dérivé bromé (III) que l'on fait réagir avec la benzyl-4 pipéridine dans un solvant tel que l'éthanol en présence d'une base, telle que le carbonate de sodium; pour obtenir le composé (IV) que l'on réduit par exemple à l'aide de borohydrure de potassium, en milieu acide tel que l'acide acétique.

L'exemple suivant illustre l'invention.

Les analyses et les spectres IR et RMN confirment la structure des composés.

*Schéma réactionnel*

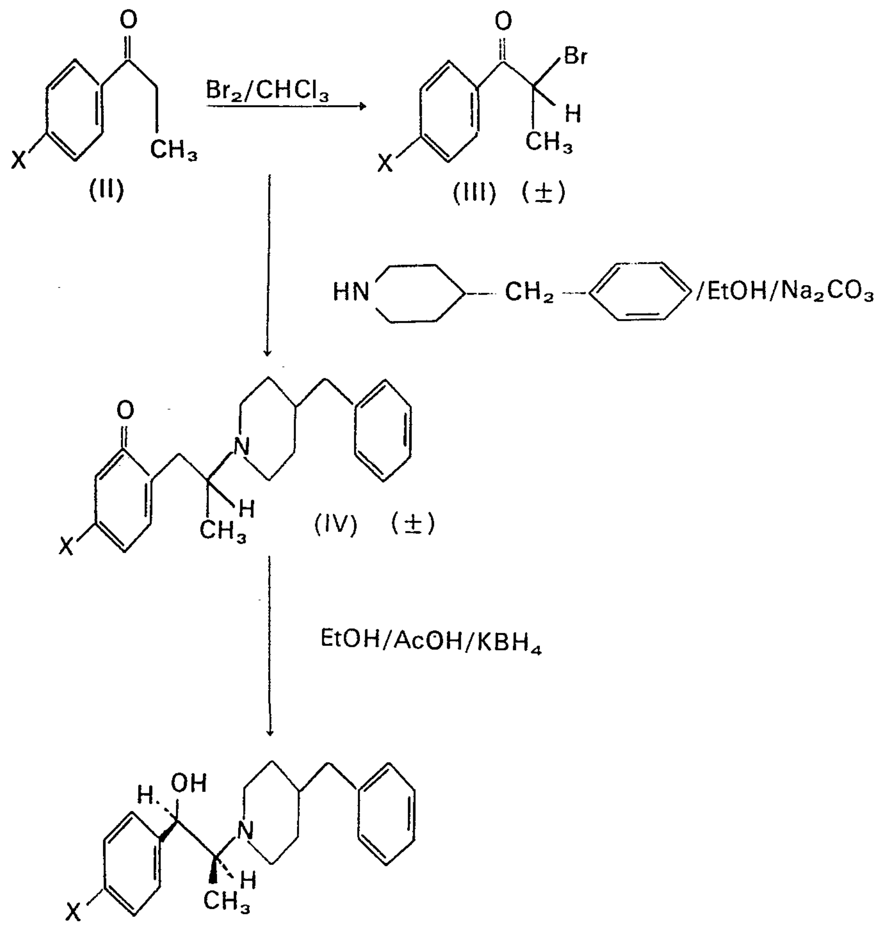


(II)

(III) (±)

(IV) (±)

(I)

(±)erythro

*Exemple:*

*(Benzyl-4 pipéridino)-2 (chloro-4 phényl)-1-propanol-1 et son chlorhydrate [sous forme (±)érythro]*

1. Bromo-2 chloro-4' propiophénone.

On ajoute à 16,8 g (0,1 mol) de chloro-4 propiophénone en solution dans 100 ml de $CHCl_3$, en présence d'$AlCl_3$, lentement, goutte à goutte, une solution de 15,9 g (0,1 mol) de brome dans 20 ml de $CHCl_3$. On agite pendant une nuit. Après filtration sur tampon de verre et après évaporation du solvant, on lave le résidu cristallisé avec de l'éther de pétrole. Le composé fond à 75° C.

2. Chloro-4' (benzyl-4 pipéridino)-2 propiophénone.

On ajoute à une solution de 20,8 g (0,084 mol) du composé obtenu précédemment dans 100 ml d'éthanol, 14,7 g (0,084 mol) de benzyl-4 pipéridine; puis 10 g (0,084 mol) de carbonate de

sodium. On chauffe à la température du reflux pendant 2 h. On refroidit à l'aide d'un bain de glace. On verse le mélange réactionnel sur 500 ml de glace et on ajoute 250 ml de toluène. On agite vivement et laisse décanter. On lave la solution organique et on l'évapore. Le composé cristallise. Après recristallisation dans 100 ml d'éthanol il fond à 92° C.

3. (Benzyl-4 pipéridino)-2 (chloro-4 phényl)-1 propanol-1 et son chlorhydrate [sous forme (±)érythro].

A une solution de 4,3 g (0,0125 mol) du composé obtenu précédemment dans 40 ml d'éthanol, on ajoute 20 ml d'acide acétique. On plonge le mélange réactionnel dans un bain de glace et on ajoute, peu à peu, tout en agitant, 3 g de $KBH_4$. On agite 1 h à la température ambiante. On ajoute 100 ml d'un mélange d'eau et de glace et 100 ml de $CH_2Cl_2$. On alcalinise avec 15 ml d'ammoniaque concentré. On agite puis décante la phase organique. On extrait la phase aqueuse avec 60 ml de $CH_2Cl_2$. On lave les phases organiques et les évapore.

Le résidu cristallise et est séché par entraînement azéotropique avec du toluène. On reprend le résidu avec 50 ml d'éthanol. On chauffe la suspension à la température du reflux et ajoute 15 ml d'éthanol saturé de gaz chlorhydrique. On fait refroidir le mélange réactionnel dans un bain de glace et filtre. On fait recristalliser le composé obtenu dans 60 ml d'éthanol.

F = 240-246° C.

Dans le tableau suivant sont représentés les composés préparés à titre d'exemples.

| Composé | X | F (°C) |
|---|---|---|
| 1 | Cl | 240-246 |
| 2 | F | 239-240 |
| 3 | Br | 250 (déc.) |

Les composés de l'invention ont été soumis à des essais pharmacologiques.

La toxicité (dose létale 50, $DL_{50}$) des composés a été déterminée chez des souris de souche CDI par méthode graphique.

La $DL_{50}$ est égale ou inférieure à 1000 mg/kg par voie i.p. et elle est supérieure à 1000 mg/kg par voie orale.

Les composés de l'invention ont été soumis au test de l'ischémie cérébrale complète. L'ischémie est due à un arrêt cardiaque induit par une injection intraveineuse rapide de $MgCl_2$. Dans ce test on mesure le temps de survie, c'est-à-dire l'intervalle entre le moment de l'injection de $MgCl_2$ et le dernier mouvement inspiratoire observable de chaque souris.

Ce dernier mouvement est considéré comme l'indice ultime d'une fonction du système nerveux central.

L'arrêt respiratoire apparaît approximativement 19 s après l'injection de $MgCl_2$.

Des souris mâles (Charles River CDI) sont étudiées par groupes de 10.

Les souris sont nourries et abreuvées *ad libitum* avant les essais. Le temps de survie est mesuré 10 min après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.

Les rapports entre les modifications dans le temps de survie et la dose du composé sont enregistrés graphiquement selon une courbe semi-logarithmique.

Cette courbe permet le calcul de la dose effective 3 s ($DE_{3''}$), c'est-à-dire la dose (en mg/kg) qui produit une augmentation de 3 s du temps de survie par rapport au groupe témoin de 10 souris non traitées.

Une augmentation de 3 s dans le temps de survie est à la fois significative statistiquement et reproductible.

La $DE_{3''}$ des composés de l'invention varie de 25 à 30 mg/kg par voie intrapéritonéale.

L'étude pharmacologique des composés de l'invention montre qu'ils possèdent une activité antianoxique et qu'ils peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, pour le traitement des encéphalopathies métaboliques, et pour le traitement des états dépressifs.

L'invention comprend par conséquent toutes compositions pharmaceutiques renfermant les composés et/ou leurs sels comme principes actifs, en association avec tous excipients appropriés à leur administration, en particulier par voie orale ou parentérale.

Les voies d'adminitration peuvent être les voies orale et parentérale.

La posologie quotidienne peut aller de 1 à 100 mg par voie parentérale et de 5 à 500 mg par voie orale.

**Revendications** pour les Etats contractants: BE, CH, DE, FR, GB, IT, LI, LU, NL, SE

1. Dérivés de phénéthanolamine, sous la forme (±)érythro, répondant à la formule (I)

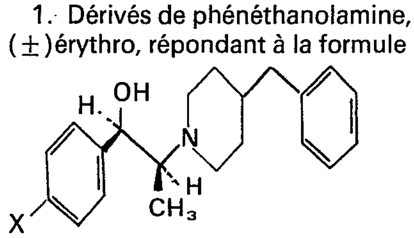

(I)

(±)erythro

dans laquelle X est un atome d'halogène, ainsi que leurs sels d'addition à des acides acceptables du point de vue pharmacologique.

2. Procédé de préparation des composés selon la revendication 1, procédé caractérisé en ce que l'on fait réagir une halogéno-4 propiophénone de formule (II)

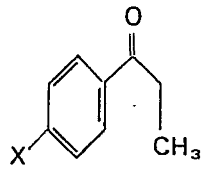

(II)

avec du brome dans un solvant tel que le chloroforme pour obtenir le dérivé bromé (III)

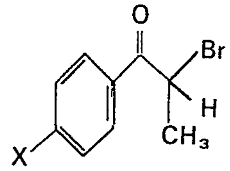

(III) (±)

que l'on fait réagir avec la benzyl-4 pipéridine dans un solvant tel que l'éthanol en présence d'une base, tel que le carbonate de sodium; pour obtenir le composé (IV)

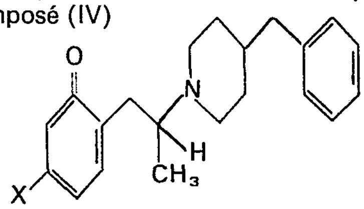

(IV) (±)

que l'on réduit par exemple à l'aide de borohydrure de potassium, en milieu acide tel que l'acide acétique.

3. Médicament caractérisé en ce qu'il contient un composé selon la revendication 1.

**Revendication** pour l'Etat contractant: AT

Procédé de préparation de dérivés de phénéthanolamine, sous la forme (±)érythro, répondant à la formule (I)

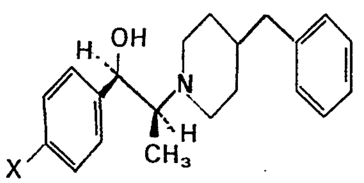

(I) (±)erythro

dans laquelle X est un atome d'halogène, ainsi que leurs sels d'addition à des acides acceptables du point de vue pharmacologique,

procédé caractérisé en ce que l'on fait réagir une halogéno-4 propiophénole de formule (II)

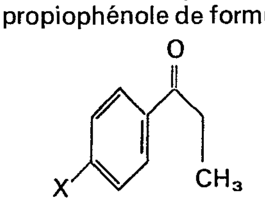

(II)

avec du brome dans un solvant tel que le chloroforme pour obtenir le dérivé bromé (III)

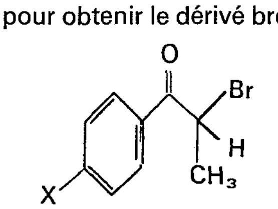

(III) (±)

que l'on fait réagir avec la benzyl-4 pipéridine dans un solvant tel que l'éthanol en présence d'une base, telle que le carbonate de sodium, pour obtenir le composé (IV)

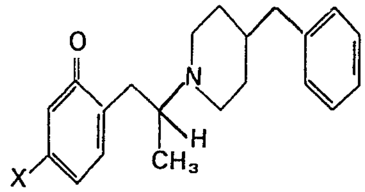

(IV) (±)

que l'on réduit par exemple à l'aide de borohydrure de potassium, en milieu acide tel que l'acide acétique.

**Claims** for the contracting States: BE, CH, DE, FR, GB, IT, LI, LU, NL, SE

1. Phenethanolamine derivatives, in the (±) erythro form, corresponding to Formula (I)

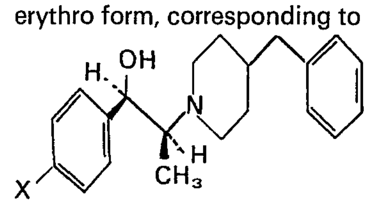

(I) (±)erythro

wherein X is a halogen atom, and their addition salts with pharmacologically acceptable acids.

2. A process for preparing the compounds according to Claim 1, characterized in that a 4-halogeno-propiophenone of Formula (II)

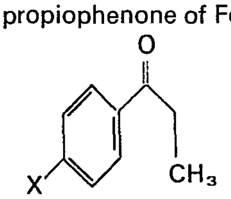

(II)

becomes reacted with bromine in a solvent such as chloroform, so as to produce the brominated derivative (III)

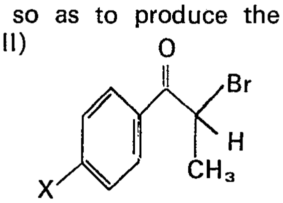

(III) (±)

which becomes reacted with 4-benzyl-piperidine in a solvent such as ethanol in presence of a base such as sodium carbonate; so as to produce compound (IV)

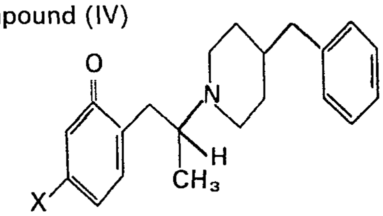

(IV) (±)

which becomes reduced, e.g. by potassium borohydride, in an acidic medium such as acetic acid.

3. A drug characterized in that it contains a compound according to Claim 1.

**Claim** for the contracting State: AT

A process for preparing phenethanolamine derivatives, in the (±) erythro form, corresponding to Formula (I)

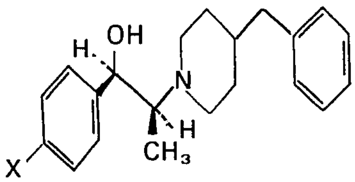

(I)

(±)erythro

wherein X is a halogen atom, and their addition salts with pharmacologically acceptable acids,

process characterized in that a 4-halogeno-propiophenone of Formula (II)

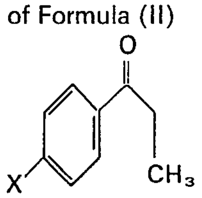

(II)

becomes reacted with bromine in a solvent such as chloroform, so as to produce the brominated derivative (III)

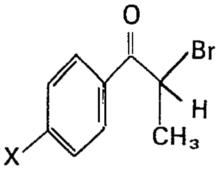

(III)
(±)

which becomes reacted with 4-benzyl-piperidine in a solvent such as ethanol in presence of a base such as sodium carbonate; so as to produce compound (IV)

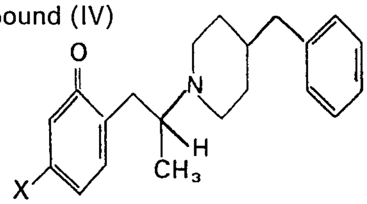

(IV)
(±)

which becomes reduced, e.g. by potassium borohydride, in an acidic medium such as acetic acid.

**Patentansprüche** für die Vertragsstaaten: BE, CH, DE, FR, GB, IT, LI, LU, NL, SE

1. Phenethanolaminderivate, in der Form (±) Erythro, entsprechend Formel (I)

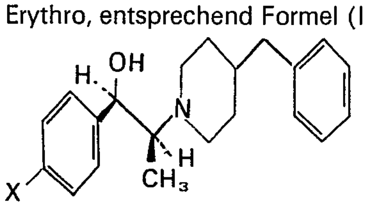

(I)

(±)erythro

worin X für ein Halogenatom steht, sowie deren Additionssalze mit pharmakologisch annehmbaren Säuren.

2. Verfahren zur Herstellung der Verbindungen nach Anspruch 1, dadurch gekennzeichnet, dass man ein 4-Halogeno-Propiophenon der Formel (II)

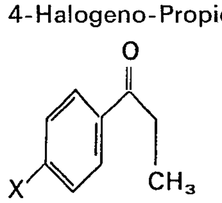

(II)

mit Brom in einem Lösungsmittel wie Chloroform versetzt, um dabei das bromierte Derivat (III) zu erhalten,

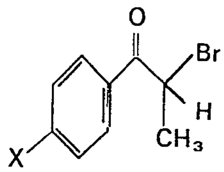

(III)
(±)

das man mit einem 4-Benzyl-Piperidin in einem Lösungsmittel wie Äthanol, in Gegenwart einer Base wie Natriumcarbonat, versetzt, um dabei die Verbindung (IV) zu erhalten,

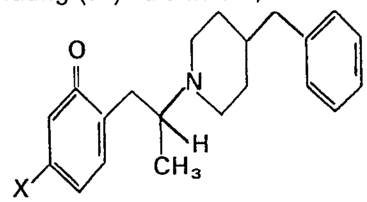

(IV)
(±)

die man z.B. mittels Kaliumborohydrid in einem säuren Medium wie Essigsäure reduziert.

3. Arzneimittel, dadurch gekennzeichnet, dass es eine Verbindung nach Anspruch 1 enthält.

**Patentanspruch** für den Vertragsstaat: AT

Verfahren zur Herstellung von Phenethanolaminderivaten, in der Form (±) Erythro, entsprechend der Formel (I)

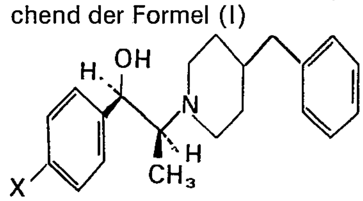

(I)

(±)erythro

worin X für ein Halogenatom steht, sowie deren Additionssalze mit pharmakologisch annehmbaren Säuren,

dadurch gekennzeichnet, dass man ein 4-Halogeno-Propiophenon der Formel (II)

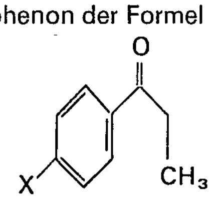

(II)

mit Brom in einem Lösungsmittel wie Chloroform versetzt, um dabei das bromierte Derivat (III) zu erhalten,

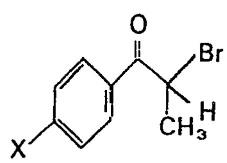

(III)
(±)

das man mit einem 4-Benzyl-Piperidin in einem Lösungsmittel wie Äthanol, in Gegenwart einer Base wie Natriumcarbonat versetzt, um dabei die Verbindung (IV) zu erhlaten,

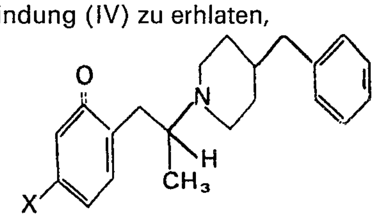

(IV)
(±)

die man z.B. mittels Kaliumborohydrid in einem säuren Medium wie Essigsäure reduziert.